# EUROPÄISCHE PATENTSCHRIFT

(11) **EP 0 882 985 B1**
(45) Veröffentlichungstag und Bekanntmachung des Hinweises auf die Patenterteilung: **28.08.2002**
(21) Anmeldenummer: 98108224.1
(22) Anmeldetag: 06.05.1998
(51) Int. Cl.: G01N 33/53, G01N 33/531, G01N 33/543, G01N 33/68, G01N 33/569

(54) **Immunchemische Bestimmung von multivalenten Analyten**
Immunochemical determination of multivalent analytes
Détermination immunochimique d'analytes multivalents

(30) Priorität: 04.06.1997 DE 19723463
(43) Veröffentlichungstag der Anmeldung: 09.12.1998
(73) Patentinhaber: Dade Behring Marburg GmbH, 35001 Marburg (DE)
(72) Erfinder: Brust, Stefan, Dr., 35041 Marburg (DE); Hauser, Hans-Peter, Dr., 35041 Marburg (DE); Knapp, Stefan, Dr., 35043 Marburg (DE); Peters, Helmut, Dr., 35043 Marburg (DE)

(56) Entgegenhaltungen:
- EP-A- 0 187 041
- EP-A- 0 307 149
- US-A- 5 496 934

## Beschreibung

Die vorliegende Erfindung betrifft ein immunchemisches Verfahren zum qualitativen oder quantitativen Nachweis eines Analyten, worin ein erster und zweiter Bindungspartner mit dem Analyten in Kontakt gebracht werden und die Bindung der Bindungspartner an den Analyten mittels bekannter Verfahren detektiert wird. Erfindungsgemäß werden beide Bindungspartner gentechnisch im gleichen Wirt unter Verwendung unterschiedlicher Vektoren (V1 und V2) zur Expression von Fusionsproteinen hergestellt, wobei der erste Bindungspartner als Fusionsprotein F1 im Vektor V1 und der zweite Bindungspartner als Fusionsprotein F2 im Vektor V2 exprimiert wird. Der Vorteil des erfindungsgemäßen Verfahrens, das auch zur gleichzeitigen Bestimmung mehrerer Analyten eingesetzt werden kann, liegt in seiner gegenüber bekannten Verfahren verbesserten Sensitivität und Spezifität.

Übliche immunologische Verfahren zur Diagnose von Erkrankungen, die mit der Bildung von spezifischen Antikörpern gegen Krankheitsauslöser, wie beispielsweise Viren, Bakterien, Parasiten, Allergene, Autoantigene oder bestimmte Arzneimittel einhergehen, beruhen auf der Fähigkeit dieser Antikörper zur Komplexbildung mit antigenen Strukturen des Krankheitsauslösers.

Bei bestimmten Immunoassay-Verfahren wird eine auf den Gehalt, beispielsweise an spezifischen Antikörpern (Analytantikörpern) zu prüfende Probe mit antigenen Strukturen der Krankheitsauslöser in Kontakt gebracht, wobei diese antigenen Strukturen an geeigneten bekannten Trägermaterialien immobilisiert sind. In der Probe enthaltene Analytantikörper werden als Immunkomplex an die an den Trägermaterialien immobilisierten antigenen Strukturen des Krankheitsauslösers gebunden und nachgewiesen. Zum Nachweis können Nachweisantikörper bzw. andere spezifische Rezeptoren (beispielsweise Protein A), die zur Komplexbindung mit dem Analytantikörper der Probe befähigt sind oder der Analyt selbst verwendet werden.

Das Nachweisreagenz trägt in der Regel eine Markierung, welche die Menge des gebundenen Antikörpers meßtechnisch erfaßbar macht. Gebräuchliche Markierungen sind beispielsweise: radioaktive Isotope, Enzyme, fluoreszierende, phosphoreszierende oder lumineszierende Substanzen, Substanzen mit stabilen ungepaarten Elektronen, Erythrozyten, Latexpartikel, Magnetpartikel und Metallsole.

Bei diesen Verfahren sind sowohl homogene wie auch heterogene (einstufige wie auch mehrstufige) Testausführungen bekannt. Jeder Verfahrensschritt wird bei der heterogenen Ausführungsform mit einem Trennprozess (Waschschritt) beendet.

Die sehr einfach durchzuführende Technik der Einschritt-Methode bei heterogenen Immunoassays ist allerdings nicht zum Nachweis aller Krankheitsmarker geeignet. Oft müssen aus technischen Gründen zweioder mehrstufige Verfahrensschritte angewandt werden.

Bekannt sind Verfahren, die man als Doppel-Antigen-Sandwich-lmmunoassays oder Antikörper-Brücken-Teste bezeichnet. Dabei werden ein oder mehrere Festphasenantigene, der oder die spezifischen nachzuweisenden Analyten und ein oder mehrere markierte Konjugatantigene miteinander in Kontakt gebracht. Bei Anwesenheit spezifischer Analyten entstehen Komplexe, die mittels der Markierung des Konjugatantigens meßtechnisch erfaßbar sind. Entscheidend für die Geschwindigkeit der Ausbildung und Festigkeit der Komplexe ist die einheitliche Präsentation des Antigens, das durch die mehrfach vorhandene antigenbindende Domäne des Analyten verbrückt wird.

Dem Fachmann sind Ausführungsformen bekannt, bei denen die verwendeten Antigene aus natürlicherweise vorkommenden prokariontischen oder eukariontischen Zellen, aus gentechnisch veränderten prokariontischen oder eukariontischen Zellen oder durch chemische Synthese gewonnen werden. Diese Ausführungsformen sind sehr sensitiv, insbesondere wenn beide Bindungspartner des Analyten aus dem gleichen Wirt gewonnen werden, da in den meisten dieser Fälle die antigene Präsentation, der Glykosilierungsgrad und die Konformation der beiden Bindungspartner sehr ähnlich sind. Von Nachteil ist jedoch bei diesen Ausführungsformen, daß wirtsspezifische Bestandteile und/oder Verunreinigungen, die sowohl in der immobiliserten als auch in der markierten Phase vorhanden sind, durch die mögliche Anwesenheit von Rezeptoren gegen diese wirtsspezifische Bestandteile und/oder Verunreinigungen zu falsch positiven Reaktionen führen können.

Dem Fachmann sind Methoden bekannt, diese Störungen durch die Zugabe von wirtsspezifischen Bestandteilen und/oder Verunreinigungen, die keine Bindungspartner oder antigen wirksame Teile davon enthalten, zu unterdrücken. Dieses Entstören gelingt aber nur bei geringgradigen unspezifischen Reaktionen. Bei starken unspezifischen Störungen, die wesentlich höhere Mengen an Entstörkomponenten erfordern, besteht jedoch der Nachteil, daß auch das spezifische Signal stark gehemmt wird.

Eine andere Methode, Störungen durch wirtsspezifische Bestandteile und/oder Verunreinigungen möglichst zu vermeiden, wird in der EP-0 307 149 offenbart. Beschrieben wird ein Doppel-Antigen-Sandwich-Enzymimmunoassay auf Basis rekombinant hergestellter Proteine, die aus unterschiedlichen Wirtsorganismen isoliert werden. Vorteilhaft ist die fehlende oder geringe Störung durch homologe Verunreinigungen, nachteilig ist jedoch die unterschiedliche Faltung und Präsentation der beiden verwendeten Rezeptoren R1 und R2 in den unterschiedlichen Wirtsorganismen. Dies führt zu einer geringeren spezifischen Reaktion mit dem nachzuweisenden Analyten und somit zur verringerten Sensitivität im Doppel-Antigen-Sandwich-Enzymimmunoassay.

Eine weitere Möglichkeit, unspezifische Störungen im Doppel-Antigen-Sandwich-Enzymimmunoassay möglichst zu vermeiden, ist der dem Fachmann bekannte Testaufbau aus rekombinant hergestelltem Protein einerseits und einem synthetisch hergestellten Peptid andererseits. Nachteile bestehen auch hier in der verringerten Sensitivität durch die unterschiedliche Faltung und Präsentation der beiden Rezeptoren R1 und R2.

Dem Fachmann sind verschiedene Möglichkeiten bekannt, rekombinante Proteine in einem Wirtssystem zur Expression zu bringen. Die das gewünschte Antigen kodierende DNA-Sequenz wird hierzu mittels bekannter Methoden (Current Protocols of Molecular Biology, Wiley Interscience, 1995, Supplement 30) in einen sogenannten Vektor kloniert, welcher zur Replikation im gewünschten Wirt befähigt ist und der zudem für eine selektierbare Eigenschaft, wie beispielsweise eine Antibiotikumsresistenz kodiert, die es ermöglicht, vektorhaltige Zellen zu selektionieren. Der Vektor sollte weiter am 5' Ende des Gens für das rekombinante Antigen eine Promotorsequenz aufweisen, die es einer Wirts-RNA-Polymerase oder einer anderweitig zur Verfügung gestellten RNA-Polymerase ermöglicht, die Abschrift einer mRNA des rekombinanten Antigens zu initiieren. Idealerweise ist dieser Promotor gezielt induzierbar, wie dies beispielsweise im lac-Repressorsystem durch IPTG Zugabe erreichbar ist. Der Vektor sollte auch über eine DNA-Sequenz verfügen, die die Transkription der mRNA terminiert.

Der Translationsstart auf der mRNA kann dabei durch das Vektorsystem oder aber durch die DNA des rekombinanten Antigens selbst zur Verfügung gestellt werden. Der Fachmann unterscheidet dabei die reife Expression eines Proteins ohne Fremdanteile von der eines Fusionsproteins. Unter Fusionsprotein wird hier ein Fusionsprodukt, bestehend aus einem durch den Vektor kodierten Peptid oder Protein (Fusionsteil) und dem rekombinanten Antigen (Antigenteil) verstanden.

Der Vorteil einer reifen Expression für den Einsatz in der Diagnostik besteht darin, daß das exprimierte Antigen mit keinem Fusionsteil assoziiert ist, der unspezifische Reaktionen hervorrufen könnte. Nachteilig ist, daß die Stärke der Expression eines zum Wirt heterologen Proteins oft sehr gering ist und bei einem reifen Protein meist keine Möglichkeit einer affinitätschromatographischen Reinigung besteht.

Die Expression heterologer Proteine als Fusionsproteine mit einem im Wirt gut exprimierbaren Protein führt dagegen häufig zu höheren Expressionsraten. Idealerweise verleiht dabei der Fusionsanteil neben der höheren Expressionsrate auch die Möglichkeit zu einer affinitätschromatographischen Reinigung. Nachteilig ist jedoch, daß am Fusionsteil unerwünschte Reaktionen statfinden können, indem Substanzen mit Bindungsaffinität zum Fusionsteil an diesen binden.

Daher werden mit beiden Expressionsverfahren in der Praxis häufig Proteinpräparationen erhalten, die in diagnostischen Testsystemen unspezifische Reaktionen verursachen: entweder aufgrund der Anwesenheit eines Fusionsteils oder, in Abwesenheit eines Fusionsteils, aufgrund des Fehlens eines effektiven, beispielsweise affinitätschromatigraphischen Reinigungsverfahrens für das Antigen.

Vorliegender Erfindung lag daher die Aufgabe zugrunde, die Leistungsfähigkeit diagnostischer Testsysteme, welche die Verwendung gentechnisch hergestellter Bindungspartner beinhalten, zu verbessern.

Überraschend wurde festgestellt, daß es möglich ist, ein immunchemisches Testsystem von hoher Spezifität und hoher nutzbarer Sensitivität aufzubauen, wenn bei Einsatz zweier oder mehrerer rekombinant hergestellter Bindungspartner, diese im gleichen Wirt unter Verwendung verschiedener Vektoren zur Expression von Fusionsproteinen dargestellt werden. Hinsichtlich Spezifität und nutzbarer Sensitivität ist ein solches System einer bekannten Ausführungsform deutlich überlegen, bei welcher zwei oder mehr Bindungspartner im gleichen Wirt und unter Verwendung identischer Vektoren zur Expression von Fusionsproteinen dargestellt werden. Beispielsweise hat sich herausgestellt, daß ein Doppel-Antigen-Sandwich-Enzymimmunoassay bei Verwendung zweier Bindungspartner F1 und F2, welche mittels verschiedener Vektoren zur Expression von Fusionsproteinen im gleichen Wirt hergestellt wurden, deutlich sensitiver und spezifischer ist als ein Doppel-Antigen-Sandwich-Enzymimmunoassay, bei dem F1 und F2 mit dem gleichen Vektor zur Expression von Fusionsproteinen und im gleichen Wirt hergestellt wurden.

Der nachzuweisende Analyt im Sinne vorliegender Erfindung kann beispielsweise ein durch einen Krankheitsauslöser oder einen Impfstoff induzierter Antikörper, ein Multi-Hapten oder ein multivalentes Protein, beispielsweise der Krankheitsauslöser selbst sein.

Die gestellte Aufgabe wird daher durch Bereitstellung eines diagnostischen Verfahrens gelöst, welches die vorteilhafte Verwendung gentechnisch hergestellter Bindungspartner bei gleichzeitig ausgezeichneter diagnostischer Leistungsfähigkeit ermöglicht. Das erfindungsgemäße Verfahren ermöglicht die Verwendung von Fusionsproteinen, ohne daß damit die oben geschilderten Nachteile hinsichtlich der Testspezifität verbunden sind.

Gegenstand der Erfindung ist somit ein immunchemisches Verfahren zum qualitativen oder quantitativen Nachweis eines Analyten in einer Probe, worin ein erster und zweiter Bindungspartner mit dem Analyten in Kontakt gebracht werden und die Bindung der Bindungspartner an den Analyten mittels bekannter Verfahren detektiert wird, dadurch gekennzeichnet, daß beide Bindungspartner gentechnisch im gleichen Wirt unter Verwendung unterschiedlicher Vektoren (V1 und V2) zur Expression von Fusionsproteinen hergestellt werden, wobei der erste Bindungspartner als Fusionsprotein F1 im Vektor V1 und der zweite Bindungspartner als Fusionsprotein F2 im Vektor V2 exprimiert wird.

Bevorzugt ist dieser Wirt ein Bakterium oder eine Hefe.

Eine besonders bevorzugte Ausführungsform ist die Verwendung von E. coli als Wirtsorganismus.

Bevorzugte Analyten sind Antikörper, besonders bevorzugte Analyten sind Antikörper gegen Viren, Bakterien und Parasiten.

Dem Fachmann ist bekannt, daß immunchemische Verfahren, wie oben beschrieben, zur gleichzeitigen Bestimmung von unterschiedlichen Analyten, wie z.B. HIV 1 und HIV 2 oder HIV 1 und/oder HIV 2 und HCV, eingesetzt werden können. Solche Ausführungsformen sind hiermit auch eingeschlossen.

Bindungspartner im Sinne vorliegender Erfindung sind spezifische Bindungspartner, die eine oder mehr als eine bioaffine Bindungsstelle zum Analyt aufweisen. Bindungspartner oder Komponenten dieser Bindungspartner können direkt oder über bioaffine Wechselwirkungen an die Festphase gebunden werden. Als Festphasen werden hierbei bevorzugterweise Mikrotitrationsplatten, Magnetpartikel, Latexpartikel oder matrixchemische Testelemente, wie z.B. Fasern oder Membranen enthaltende Testmodule verwendet. Die Bindungspartner oder einzelne Komponenten dieser Bindungspartner können auch direkt markiert sein (Konjugat) oder über bioaffine Wechselwirkungen an eine Markierung gebunden werden.

Dem Fachmann sind Methoden zur Herstellung solcher Konjugate, die aus dem Bindungspartner und einem Label bestehen bekannt. Label können Enzyme (beispielsweise Peroxidase, Alkalische Phosphatase, Galaktosidas, Urease) oder Haptene (beispielsweise Biotin, Digoxigenin) oder fluorenszente Verbindungen (beispielsweise FITC) oder lumineszente Verbindungen (beispielsweise Acridiniumester, Metall-chelat-Komplexe) oder Metallsole (beispielsweise Gold) oder Partikel (beispielsweise Latex) sein. Solche Konjugate können, unter Erhalt der bioaffinen Funktion ihrer Ausgangsmaterialien, beispielsweise durch Verknüpfung mittels chemischer Reagenzien oder durch bioaffine Wechselwirkung hergestellt werden. Es können auch Hybridmoleküle durch chemische Synthese, die Hybridoma-Technik oder gentechnologische Methoden erzeugt werden.

Außerdem ist Gegenstand der Erfindung ein Reagenz zur Verwendung im oben genannten Verfahren.

Kombinationen von Bindungspartnern zum spezifischen Nachweis von Analyten sind:
- F1:: Fusionsprotein aus Wirtssytem A, Vektor zur Expression von Fusionsproteinen V1 und gereinigt nach Methode M1.
- F2:: Fusionsprotein aus Wirtssytem A, Vektor zur Expression von Fusionsproteinen V2 und gereinigt nach Methode M1 oder M2.

Bevorzugte Kombinationen von Bindungspartnern zum spezifischen Nachweis von Analyten sind:
- F1:: Fusionsprotein aus Wirtssytem A, Vektor zur Expression von Fusionsproteinen V1 und gereinigt nach Methode M1.
- F2:: Fusionsprotein aus Wirtssytem A, Vektor zur Expression von Fusionsproteinen V2 und gereinigt nach Methode M2.

Besonders bevorzugte Kombinationen von Bindungspartnern zum spezifischen Nachweis von Analyten sind:
- F1:: in physiologischen Puffern unlösliches Fusionsprotein aus Wirtssytem A, Vektor zur Expression von Fusionsproteinen V1 und gereinigt nach Methode M1.
- F2:: In physiologischen Puffern lösliches Fusionsprotein aus Wirtssytem A, Vektor zur Expression von Fusionsproteinen V2 und gereinigt nach Methode M2.

Wirtssyteme im Sinne vorliegender Erfindung sind Organismen, die in der Lage sind, mit einem Vektor zur Expression von Fusionsproteinen transformiert und danach auf Vorhandensein des Vektors selektiert zu werden, diesen zu replizieren und das im Vektor klonierte rekombinante Gen zu transskribieren und zu translatieren.

Vektoren zur Expression von Fusionsproteinen im Sinne der Erfindung sind zur Replikation im geeigneten Wirt befähigte Nukleinsäuren, welche für eine selektierbare Eigenschaft kodieren, die die Selektion vektorhaltiger Wirtsorganismen ermöglicht. Vektoren zur Expression von Fusionsproteinen weisen idealerweise induzierbare Promotoren auf hinter welche die Gene für die rekombinanten Antigene kloniert werden können und daher die gezielte Transkription und Translation des rekombinanten Antigens ermöglichen.

Bevorzugte Vektoren zur Expression von Fusionsproteinen im Sinne der vorliegenden Erfindung exprimieren das Zielprotein in Fusion mit einem im jeweiligen Wirt gut exprimierbaren Anteil.

Besonders bevorzugte Vektoren zur Expression von Fusionsproteinen im Sinne der vorliegenden Erfindung exprimieren das Zielprotein in Fusion mit zwei unterschiedlichen im jeweiligen Wirt gut exprimierbaren Anteilen.

Ganz besonders bevorzugte Vektoren zur Expression von Fusionsproteinen im Sinne der vorliegenden Erfindung exprimieren das Zielprotein in Fusion mit zwei unterschiedlichen im jeweiligen Wirt gut exprimierbaren Anteilen, wobei der jeweilige Fusionsanteil das jeweilige Fusionsprotein unterschiedlichen Reinigungsmethoden zugänglich macht.

Reinigungsmethoden im Sinne der Erfindung sind Methoden, die es erlauben, das im Wirtssystem exprimierte Zielprotein in reiner Form darzustellen. Werden keine sezernierten Antigene hergestellt, muß der Wirtsorganismus zunächst aufgeschlossen werden. Dem Fachmann sind hierzu verschiedene Methoden bekannt, wie beispielsweise das Erzeugen extremer Druckunterschiede in einer French Press. Eine Reinigung des Antigens kann dann auf dem Fachmann bekannten Methoden beruhen, die sich die physikochemischen Eigenschaften des Antigens, wie beispielsweise Löslichkeit, isoelektrischer Punkt, Ladung, Größe und elektrophoretische Beweglichkeit zunutze machen. Eine besonders starke Anreicherung des Zielproteins läßt sich erreichen, wenn sich das Zielprotein spezifisch und reversibel an eine Matrix binden läßt, so daß es einer affinitätschromatographischen Reinigung zugänglich ist.

Das erfindungsgemäße Reagenz kann Verwendung finden in einer Vielzahl von Verfahren der Human- und Veterinärdiagnostik. Als Beispiele sollen ein- oder mehrstufige Teste zu Nachweis von Antikörpern verschiedener Immunglobulin-Klassen gegen Strukturmerkmale von Viren (beispielsweise Viren der Hepatitis A, B und C, sowie verschiedener HIV-Typen), von bakteriellen und parasitären Erregern sowie von allergischen Erkrankungen angeführt werden. Weitere Beispiele sind der Nachweis von Krankheitserregern wie Viren (beispielsweise Hepatitis B-Virus), Bakterien, Parasiten und Allergenen wie auch von Markern von Krankheiten (beispielsweise Tumormarkern) in ein- oder mehrstufigen Nachweisverfahren.

Die Erfindung wird außerdem durch die folgenden Beispiele erläutert, ohne sie jedoch darauf einzuschränken:

### Beispiel 1:

### Enzymimmunoassay zum Nachweis von HIV1-Antikörpern

### 1a) Herstellung der rekombinanten Proteine pMAL-gp41 und pSEM-gp41

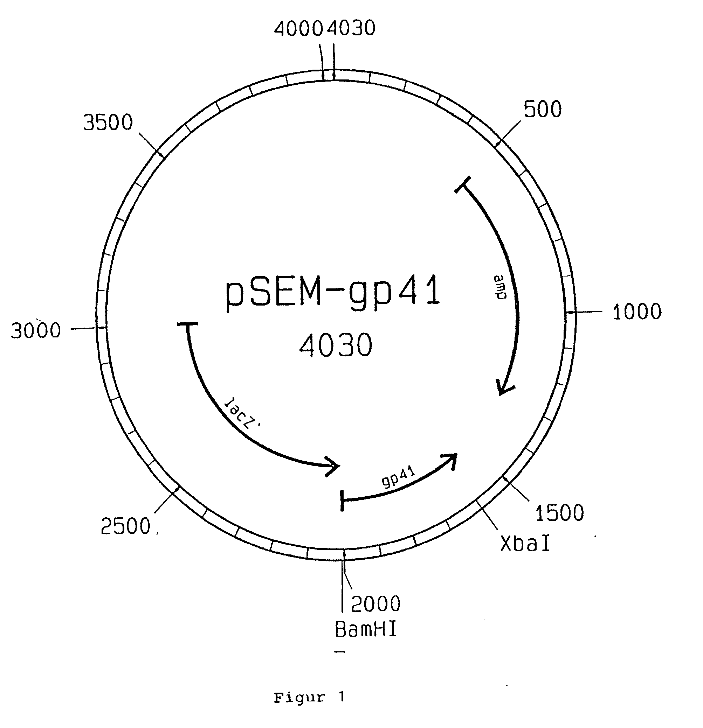

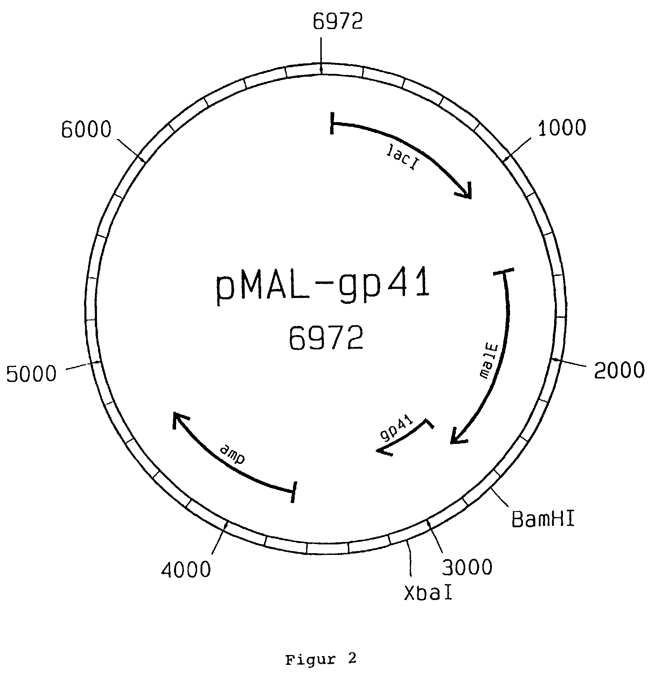

Die für die HIV-Antigene kodierende DNA wurde mittels PCR hergestellt. Template für die PCR war der HIV-Stamm HIV1-BH10. Das amplifizierte Fragment umfaßte dabei bp7209-bp7508 (Seq.ld. 1). Durch Einführung der Restriktionsschnittstellen BamHI am 5' Ende und Xbal am 3'Ende des Fragments konnte die Klonierung in die Expressionsvektoren pMAL-c2™ (New England Biolabs) und pSEM (Knapp et al., Biotechniques, 1990, Vol 8, no.3, p280-281) durchgeführt werden. Die Expression und Reinigung der rekombinanten Proteine erfolgte im Fall von pSEM-gp41 (figure 1) durch differentielle Harnstoffextraktion bei 7M Harnstoff aus den Inclusion Bodies und anschließender gelchromatographischer Reinigung, und im Fall von pMAL-gp41 (figure 2) durch Affinitätschromatographie entsprechend der Angaben des Herstellers. Beide Proteine werden auf eine Konzentration von 1 g/l eingestellt. Das pMAL-gp41 -Protein liegt in 50 mM Natriumcarbonat, pH 8.0 , das pSEM-gp41 -Protein in 50 mM Natriumcarbonat, 6 M Harnstoff, pH 8.0 vor.

### 1b) Herstellung der Festphase I (Erfindungsgemäßes System)

Mikrotitrationsplatten Typ B (Fa. Nunc, Roskilde, Dänemark) werden mit 100 µl je Vertiefung Beschichtungslösung (600 µg/l rekombinantes pSEM-gp41 [Herstellung nach Beispiel 1a in 50 mM Natriumcarbonat-Puffer, pH 9.5) für 24 Stunden bei 4°C inkubiert. Die Vertiefungen der Mikrotitrationsplatten werden dann dreimal mit je 300 µl Waschlösung (50 mM Tris, 0.1% Tween 20, pH 7.2) gewaschen. Die über Kieselgel getrockneten Mikrotitrationsplatten sind unter Luftausschluß etwa 1 Jahr stabil.

### 1c) Herstellung der Festphase II (Referenzsystem)

Mikrotitrationsplatten Typ B (Fa. Nunc, Roskilde, Dänemark) werden mit 100 µl je Vertiefung Beschichtungslösung (600 µg/l rekombinantes pMAL-gp41 [Herstellung nach Beispiel 1a] in 50 mM Natriumcarbonat-Puffer, pH 9.5) für 24 Stunden bei 4°C inkubiert. Die Vertiefungen der Mikrotitrationsplatten werden dann dreimal mit je 300 µl Waschlösung (50 mM Tris, 0.1% Tween 20, pH 7.2) gewaschen. Die über Kieselgel getrockneten Mikrotitrationsplatten sind unter Luftausschluß etwa 1 Jahr stabil.

### 1d) Herstellung des Konjugates

10 mg rekombinantes pMAL-gp41 [Herstellung nach Beispiel 1a] werden mit einem 10fach molaren Überschuß an N-γ-Maleimidobutyrylsuccinimid versetzt und 1 Stunde bei Raumtemperatur inkubiert. Das nicht umgesetzte heterobifunktionelle Reagenz wird durch Gelfiltration (Sephadex G-25) mit 100 mM Natriumphosphat, 5 mM Nitriloessigsäure, pH 6.0 abgetrennt.

10 mg Meerrettich-Peroxidase (Boehringer Mannheim, Mannheim, BRD) werden in 10 ml 10 mM Natriumphosphat, 100 mM Kochsalz, pH 8.0 mit 100fach molarem Überschuß an 2-Iminothiolan 1 Stunde bei Raumtemperatur inkubiert. Anschließend wird freies Modifierungsreagenz durch Gelfiltration (Sephadex G-25) mit 100 mM Natriumphosphat, 5 mM Nitriloessigsäure, pH 6.0 entfernt.

Beide Eluate (SH-aktivierte Peroxidase und Maleimid-modifiziertes HIV 1-Protein) werden vereinigt und über Nacht bei Raumtemperatur inkubiert. Nach Abstoppen mit 1/10 Vol 100 mM N-Ethyl-Maleimid wird das Konjugat durch Gelfiltration (Sephadex G-25) von nicht abreagiertem HIV 1- Protein gereinigt. Nach Ankonzentrieren (2 mg/ml) wird das Konjugat bei -20°C gelagert.

### 1e) Enzymimmunoassay zum Nachweis von HIV 1-Antikörpern

Ein Enzymimmunassay zum Nachweis von Anti-HIV 1 wird wie folgt durchgeführt:
In die Vertiefungen der nach Beispiel 1a und 1b hergestellten Mikrotitrationsplatten werden 25 µl Probenpuffer (0.3 M Tris/HCl, 1% Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 125 µl des nach Beispiel 1c hergestellten Konjugates (1:1000 in 0.1 M Tris/HCl, 1 mM Glycin, 0.2 % Albumin, 0.4 % Pluronic F64, pH 8.1) einpipettiert. Mit 4 weiteren Waschschritten wird die 30minütige Inkubation (+37°C) beendet. Die gebundene Peroxidase-Aktivität, die direkt mit der Anzahl der gebundenen Anti-HIV 1-Antikörper korreliert, wird durch Zugabe von H₂O₂/ Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt.
Anti-HIV 1 positive, normale anti-HIV negative sowie ausgewählte im Referenzsystem falsch positive anti-HIV negative Seren wurden sowohl im Referenzsystem wie auch im erfindungsgemäßen Enzymimmunoassay untersucht.

Die Resultate (Extinktionseinheiten) der Untersuchung finden sich in der Tabelle 1.

**Tabelle 1:**

| **Proben-Bezeichnung** | **Anti-HIV Status** | **Verdünnung** | **Referenzsystem** | **Erfindungsgemäßes System** |
|---|---|---|---|---|
| **Negative Kontrolle** | Negativ | nativ | 0,098 | 0,103 |
| **Positive Kontrolle** | Positiv | 1:4000 | 1,575 | 1,412 |
| **9111/39** | Positiv | 1:100 | 1,211 | 1,653 |
| **9111/39** | Positiv | 1:200 | 0,520 | 0,886 |
| **9111/46** | Positiv | 1:50 | 0,528 | 0,927 |
| **9111/46** | Positiv | 1:100 | 0,310 | 0,489 |
| **9205/19** | Positiv | 1:8000 | 0,906 | 0,877 |
| | | | | |
| **BS 17-1** | Negativ | nativ | 0,058 | 0,064 |
| **BS 17-2** | Negativ | nativ | 0,071 | 0,070 |
| **BS 17-3** | Negativ | nativ | 0,066 | 0,051 |
| **BS 17-4** | Negativ | nativ | 0,066 | 0,059 |
| **BS 17-5** | Negativ | nativ | 0,082 | 0,074 |
| | | | | |
| **BS 1-5** | Negativ | nativ | **1,876** | 0,089 |
| **BS 1-17** | Negativ | nativ | **2,077** | 0,078 |
| **BS 1-33** | Negativ | nativ | **>2,5** | 0,075 |
| **BS 1-59** | Negativ | nativ | **>2,50** | 0,091 |

Deutliche Unterschiede in der Signalbildung der beiden Testsystemen ist insbesondere bei Anti-HIV negativen Proben zu erkennen. Anti-HIV positive Seren reagieren in beiden Testsystemen vergleichbar.

### Beispiel 2:

### Enzymimmunoassay zum Nachweis von T. pallidum Antikörpern

### 2a) Herstellung der rekombinanten Antigene

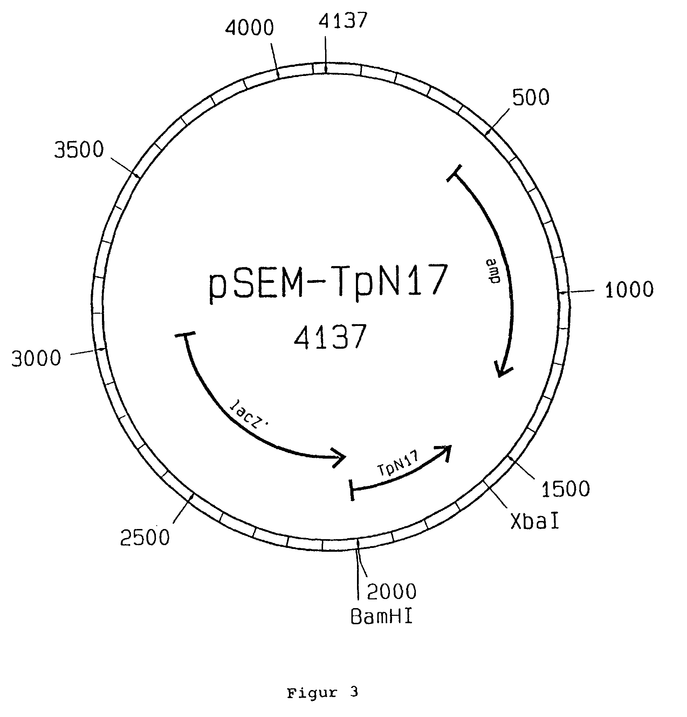

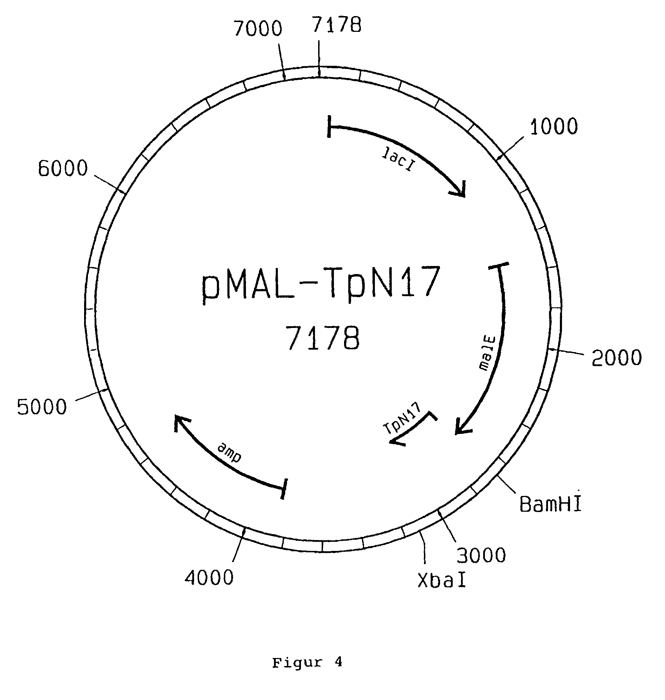

Die für die T. pallidum Antigene kodierende DNA wurde mittels PCR hergestellt. Template für die PCR war dabei genomische DNA des T. pallidum Stamms Nichols. Die PCR Primer wurden dergestalt entworfen, daß der gesamte offene Leserahmen des ProteinsTpN17 (Seq.-Id. 2) amplifiziert und je eine BamHI-Schnittstelle am 5'Ende und eine Xbal Schnittstelle am 3' Ende des Amplifikats eingeführt wurde. Mittels der Restriktionsendonukleasen BamHI und XbaI konnte dann die Klonierung in die Expressionsvektoren pMAL-c2™ (New England Biolabs) und pSEM (Knapp et al., Biotechniques, 1990, Vol 8, no.3, p280-281) durchgeführt werden. Die Expression und Reinigung der rekombinanten Proteine erfolgte im Fall von pSEM-TpN17 (figure 3) durch differentielle Harnstoffextraktion bei 7M Harnstoff aus den Inclusion Bodies und anschließender gelchromatographischer Reinigung, im Fall von pMAL-TpN17 (figure 4) durch Affinitätschromatographie entsprechend der Angaben des Herstellers. Die Proteine werden auf eine Konzentration von 1 g/l eingestellt. Das pMAL-TpN17 -Protein liegt in 50 mM Natriumcarbonat, pH 8.0, das pSEM-TpN17 - Protein in 50 mM Natriumcarbonat, 6 M Harnstoff, pH 8.0 vor.

### 2b) Herstellung der Festphase I (Erfindungsgemäßes System)

Mikrotitrationsplatten Typ B (Fa. Nunc, Roskilde, Dänemark) werden mit 100 µl je Vertiefung Beschichtungslösung (3mg/l rekombinantes pSEM-Tpn17 [Herstellung nach Beispiel 2a] in 50 mM Natriumcarbonat-Puffer, pH 9.5) für 24 Stunden bei 4°C inkubiert. Die Vertiefungen der Mikrotitrationsplatten werden dann dreimal mit je 300 µl Waschlösung (50 mM Tris, 0.1% Tween 20, pH 7.2) gewaschen. Die über Kieselgel getrockneten Mikrotitrationsplatten sind unter Luftausschluß etwa 1 Jahr stabil.

### 2c) Herstellung der Festphase II (Referenzsystem)

Mikrotitrationsplatten Typ B (Fa. Nunc, Roskilde, Dänemark) werden mit 100 µl je Vertiefung Beschichtungslösung (3 mg/l rekombinantes pMAL-TpN17 [Herstellung nach Beispiel 2a] in 50 mM Natriumcarbonat-Puffer, pH 9.5) für 24 Stunden bei 4°C inkubiert. Die Vertiefungen der Mikrotitrationsplatten werden dann dreimal mit je 300 µl Waschlösung (50 mM Tris, 0.1% Tween 20, pH 7.2) gewaschen. Die über Kieselgel getrockneten Mikrotitrationsplatten sind unter Luftausschluß etwa 1 Jahr stabil.

### 2d) Herstellung des Konjugates

10 mg rekombinantes pMAL-TpN17 [Herstellung nach Beispiel 2a] werden mit einem 10fach molaren Überschuß an N-γ-Maleimidobutyrylsuccinimid versetzt und 1 Stunde bei Raumtemperatur inkubiert. Das nicht umgesetzte heterobifunktionelle Reagenz wird durch Gelfiltration (Sephadex G-25) mit 100 mM Natriumphosphat, 5 mM Nitriloessigsäure, pH 6.0 abgetrennt.

2 mal 10 mg Meerrettich-Peroxidase (Boehringer Mannheim, Mannheim, BRD) werden in 10 ml 10 mM Natriumphosphat, 100 mM Kochsalz, pH 8.0 mit 100fach molarem Überschuß an 2-lminothiolan 1 Stunde bei Raumtemperatur inkubiert. Anschließend wird freies Modifierungsreagenz durch Gelfiltration (Sephadex G-25) mit 100 mM Natriumphosphat, 5 mM Nitriloessigsäure, pH 6.0 entfernt.

Die Eluate (SH-aktivierte Peroxidase und Maleimid-modifiziertes T. pallidum -Protein) werden vereinigt und über Nacht bei Raumtemperatur inkubiert. Nach Abstoppen mit 1/10 Vol 100 mM N-Ethyl-Maleimid wird das Konjugat durch Gelfiltration (Sephadex G-25) von nicht abreagiertem T. pallidum Protein gereinigt. Nach Ankonzentrieren (2 mg/ml) werden die Konjugate bei -20°C gelagert.

### 2e) Enzymimmunoassay zum Nachweis von T.pallidum-Antikörpern

Ein Enzymimmunassay zum Nachweis von Anti-T.pallidum wird wie folgt durchgeführt:
In die Vertiefungen der nach Beispiel 2b und 2c hergestellten Mikrotitrationsplatten werden 25 µl Probenpuffer (0.3 M Tris/HCl, 1% Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 125 µl der nach Beispiel 1c hergestellten Konjugate (1:1000 in 0.1 M Tris/HCl, 1 mM Glycin, 0.2 % Albumin, 0.4 % Pluronic F64, pH 8.1) einpipettiert. Mit 4 weiteren Waschschritten wird die 30minütige Inkubation (+37°C) beendet. Die gebundene Peroxidase-Aktivität, die direkt mit der Anzahl der gebundenen Anti- T.pallidum -Antikörper korreliert, wird durch Zugabe von H₂O₂/ Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt.

Anti-T.pallidum positive sowie anti-T.pallidum negative Seren wurden sowohl im Referenzsystem wie auch im erfindungsgemäßen Enzymimmunoassay untersucht.

Die Resultate (Extinktionseinheiten) der Untersuchung finden sich in der Tabelle 2.

**Tabelle 2:**

| **Proben-Bezeichnung** | **Anti- T.pallidum Status** | **Ver- dünnung** | **Erfindungsgemäßes System** | **Referenzsystem** |
|---|---|---|---|---|
| **Boston Biomedical BM69640** | positiv | nativ | >2,5 | >2,5 |
| **Boston Biomedical BM69631** | positiv | nativ | >2,5 | >2,5 |
| **Boston Biomedical BM69232** | positiv | nativ | >2,5 | >2,5 |
| **Boston Biomedical BM69641** | positiv | nativ | >2,5 | >2,5 |
| **Boston Biomedical BM69642** | positiv | nativ | >2,5 | >2,5 |
| **Boston Biomedical BM69639** | positiv | nativ | >2,5 | >2,5 |
| **Boston Biomedical BM68378** | positiv | nativ | >2,5 | >2,5 |
| **Boston Biomedical BM69624** | positiv | nativ | >2,5 | >2,5 |
| **Boston Biomedical BM69625** | positiv | nativ | >2,5 | >2,5 |
| **Boston Biomedical BM69214** | positiv | nativ | >2,5 | >2,5 |
| **Boston Biomedical BM69577** | positiv | nativ | >2,5 | >2,5 |
| **Blutspender # 40** | negativ | nativ | 0,015 | 0,068 |
| **Blutspender # 49** | negativ | nativ | 0,033 | 0,036 |
| **Blutspender # 50** | negativ | nativ | 0,029 | 0,073 |
| **Blutspender # 51** | negativ | nativ | 0,012 | 0,050 |
| **Blutspender # 52** | negativ | nativ | 0.024 | **0,260** |
| **Blutspender # 53** | negativ | nativ | 0,090 | 0,035 |
| **Blutspender # 54** | negativ | nativ | 0,012 | 0,041 |
| **Blutspender # 55** | negativ | nativ | 0,015 | 0,066 |
| **Blutspender # 56** | negativ | nativ | 0,013 | **0,240** |
| **Blutspender # 57** | negativ | nativ | 0,019 | 0,038 |
| **Blutspender # 58** | negativ | nativ | 0,013 | 0,061 |
| **Blutspender # 59** | negativ | nativ | 0,010 | 0,035 |
| **Blutspender # 60** | negativ | nativ | 0,008 | **0,122** |
| **Blutspender # 61** | negativ | nativ | 0,013 | 0,050 |
| **Blutspender # 62** | negativ | nativ | 0,023 | 0,068 |
| **Blutspender # 63** | negativ | nativ | 0,036 | 0,067 |
| **Blutspender # 64** | negativ | nativ | 0,022 | 0,033 |
| **Blutspender # 65** | negativ | nativ | 0,017 | 0,044 |
| **Blutspender # 66** | negativ | nativ | 0,047 | 0,079 |
| **Blutspender # 67** | negativ | nativ | 0,017 | 0,056 |
| **Blutspender # 68** | negativ | nativ | 0,006 | 0,044 |
| **Blutspender # 69** | negativ | nativ | 0,005 | 0,073 |
| **Blutspender # 70** | negativ | nativ | 0,009 | 0,069 |
| **Blutspender # 71** | negativ | nativ | 0,009 | **1,637** |
| **Blutspender # 72** | negativ | nativ | 0,038 | 0,027 |
| **Blutspender # 73** | negativ | nativ | 0,008 | 0,029 |
| **Blutspender # 74** | negativ | nativ | 0,017 | 0,029 |
| **Blutspender # 75** | negativ | nativ | 0,011 | 0,336 |
| **Blutspender # 76** | negativ | nativ | 0,008 | 0,033 |
| **Blutspender # 77** | negativ | nativ | 0,008 | 0,056 |
| **Blutspender # 78** | negativ | nativ | 0,023 | 0,058 |
| **Blutspender # 79** | negativ | nativ | 0,005 | **0,118** |
| **Blutspender # 80** | negativ | nativ | 0,009 | **0,438** |
| **Blutspender # 81** | negativ | nativ | 0.041 | 0,870 |
| **Blutspender # 82** | negativ | nativ | 0.007 | 0,059 |
| **Blutspender # 83** | negativ | nativ | 0,012 | 0,029 |

Deutliche Unterschiede in der Signalbildung der beiden Testsystemen ist insbesondere bei Anti- T.pallidum negativen Proben zu erkennen. Anti-T.pallidum negative Seren reagieren z.T. im Referenzsystem nicht aber im erfindungsgemäßen System falsch positiv. Anti- T.pallidum positive Seren reagieren in beiden Testsystemen vergleichbar.
**Seq.-Id. 1:**
   - LOCUS: HIVBH102 8932 bp ss-RNA VRL 05-FEB-1992
   - DEFINITION: Human immunodeficiency virus type 1, isolate BH10, genome.
   - ACCESSION: M15654 K02008 K02009 K02010
   - KEYWORDS: TAR region; acquired immune deficiericy syridrome; env protein; gag protein; long terminal repeat (LTR); pol protein; polyprotein; proviral gene; reverse transcriptase; trans-activator.... 
TTATTGTCTGGTATAGTGCAGCAGCAGAACAATTTGCTGAGGGCTATTGAGGCGCAACAG
7209 -+---------+---------+---------+---------+---------+--------- 7268
AATAACAGACCATATCACGTCGTCGTCTTGTTAAACGACTCCCGATAACTCCGCGTTGTC
c L L S G I V Q Q Q N N L L R A I E A Q Q -

CATCTGTTGCAACTCACAGTCTGGGGCATCAAGCAGCTCCAGGCAAGAATCCTGGCTGTG
7269 -+---------+---------+---------+---------+---------+--------- 7328
GTAGACAACGTTGAGTGTCAGACCCCGTAGTTCGTCGAGGTCCGTTCTTAGGACCGACAC
c H L L Q L T V W G I K Q L Q A R I L A V -

GAAAGATACCTAAAGGATCAACAGCTCCTGGGGATTTGGGGTTGCTCTGGAAAACTCATT
7329 -+---------+---------+---------+---------+---------+--------- 7388
CTTTCTATGGATTTCCTAGTTGTCGAGGACCCCTAAACCCCAACGAGACCTTTTGAGTAA
c E R Y L K D Q Q L L G I W G C S G K L I -

TGCACCACTGCTGTGCCTTGGAATGCTAGTTGGAGTAATAAATCTCTGGAACAGATTTGG
7389 -+---------+---------+---------+---------+---------+--------- 7448
ACGTGGTGACGACACGGAACCTTACGATCAACCTCATTATTTAGAGACCTTGTCTAAACC
c C T T A V P W N A S W S N K S L E Q I W -

AATAACATGACCTGGATGGAGTGGGACAGAGAAATTAACAATTACACAAGCTTAATACAC
7449 -+---------+---------+---------+---------+---------+--------- 7508
TTATTGTACTGGACCTACCTCACCCTGTCTCTTTAATTGTTAATGTGTTCGAATTATGTG
c N N M T W M E W D R E I N N Y T S L I H -
Seq-Id.2
   - LOCUS: TRP17LPOPR 660 bp DNA BCT 16-JUN-1993 DEFINITION Treponema pallidum 17 kDa lipoprotein gene, complete cds.
   - ACCESSION: M74825
   - KEYWORDS: lipoprotein.
   - SOURCE: Treponema pallidum (strain Nichols, sub_species pallidum) DNA.
   - ORGANISM: Treponema pallidum ...

TGTGTCTCGTGCACAACCGTGTGTCCGCACGCCGGGAAGGCCAAAGCGGAAAAGGTAGAG
163 -------+---------+---------+---------+---------+---------+-- 222
ACACAGAGCACGTGTTGGCACACAGGCGTGCGGCCCTTCCGGTTTCGCCTTTTCCATCTC
a C V S C T T V C P H A G K A K A E K V E -

TGCGCGTTGAAGGGAGGTATCTTTCGGGGTACGCTACCTGCGGCCGATTGCCCGGGAATC
223 -------+---------+---------+---------+---------+---------+-- 282
ACGCGCAACTTCCCTCCATAGAAAGCCCCATGCGATGGACGCCGGCTAACGGGCCCTTAG
a C A L K G G I F R G T L P A A D C P G I -

GATACGACTGTGACGTTCAACGCGGATGGCACTGCGCAAAAGGTAGAGCTTGCCCTTGAG
283 -------+---------+---------+---------+---------+---------+-- 342
CTATGCTGACACTGCAAGTTGCGCCTACCGTGACGCGTTTTCCATCTCGAACGGGAACTC
a D T T V T F N A D G T A Q K V E L A L E -

AAGAAGTCGGCACCTTCTCCTCTTACCTATCGCGGTACGTGGATGGTACGTGAAGACGGA
343 -------+---------+---------+---------+---------+---------+-- 402
TTCTTCAGCCGTGGAAGAGGAGAATGGATAGCGCCATGCACCTACCATGCACTTCTGCCT
a K K S A P S P L T Y R G T W M V R E D G -

ATTGTCGAACTCTCGCTTGTGTCCTCGGAGCAATCGAAGGCACCGCACGAGAAAGAGCTG
403 -------+---------+---------+---------+---------+---------+-- 462
TAACAGCTTGAGAGCGAACACAGGAGCCTCGTTAGCTTCCGTGGCGTGCTCTTTCTCGAC
a I V E L S L V S S E Q S K A P H E K E L -

TACGAGCTGATAGACAGTAACTCCGTTCGCTACATGGGCGCTCCCGGCGCAGGAAAGCCT
463 -------+---------+---------+---------+---------+---------+-- 522
ATGCTCGACTATCTGTCATTGAGGCAAGCGATGTACCCGCGAGGGCCGCGTCCTTTCGGA
a Y E L I D S N S V R Y M G A P G A G K P -

TCAAAGGAGATGGCGCCGTTTTACGTGCTCAAAAAAACAAAGAAATAG
523 -------+---------+---------+---------+---------+ 570
AGTTTCCTCTACCGCGGCAAAATGCACGAGTTTTTTTGTTTCTTTATC
a S K E M A P F Y V L K K T K K * -

## Patentansprüche

1. Immunchemisches Verfahren zum qualitativen oder quantitativen Nachweis eines Analyten in einer Probe, worin ein erster und zweiter Bindungspartner mit dem Analyten in Kontakt gebracht werden und die Bindung der Bindungspartner an den Analyten mittels bekannter Verfahren detektiert wird, **dadurch gekennzeichnet, daß** beide Bindungspartner gentechnisch im gleichen Wirt unter Verwendung unterschiedlicher Vektoren (V1 und V2) zur Expression von Fusionsproteinen hergestellt werden, wobei der erste Bindungspartner als Fusionsprotein F1 im Vektor V1 und der zweite Bindungspartner als Fusionsprotein F2 im Vektor V2 exprimiert wird.

2. Verfahren gemäß Anspruch 1, **dadurch gekennzeichnet, daß** mindestens 2 Analyten qualitativ oder quantitativ nachgewiesen werden, daß je Analyt zwei Bindungspartner eingesetzt werden und daß diese beiden Bindungspartner gentechnisch als Fusionsproteine im gleichen Wirt unter Verwendung unterschiedlicher Vektoren zur Expression von Fusionsproteinen hergestellt werden, wobei Bindungspartner verschiedener Analyten in verschiedenen Wirten exprimiert werden können.

3. Verfahren gemäß Anspruch 1 oder 2, **dadurch gekennzeichnet, daß** einer oder mehrere der Bindungspartner an einer festen Phase immobilisiert sind.

4. Verfahren gemäß Anspruch 3, **dadurch gekennzeichnet, daß** es ein Doppel-Antigen-Sandwich-Immuntestverfahren ist.

5. Verfahren gemäß einem der vorhergehenden Ansprüche, **dadurch gekennzeichnet, daß** der Wirt ein eukaryotischer Organismus ist.

6. Verfahren gemäß einem der vorhergehenden Ansprüche, **dadurch gekennzeichnet, daß** der Wirt ein prokaryotischer Organismus ist.

7. Verfahren gemäß Anspruch 6, worin der Wirt ein Bakterium ist.

8. Verfahren gemäß Anspruch 7, worin das Bakterium E. coli ist.

9. Verfahren gemäß einem der vorhergehenden Ansprüche, **dadurch gekennzeichnet, daß** im Rahmen der Expression eines der Fusionsproteine F1 oder F2 weitgehend aus der löslichen Fraktion und das jeweils andere Fusionsprotein F1 oder F2 weitgehend aus der unlöslichen Fraktion gewonnen wird.

10. Verfahren gemäß einem der vorhergehenden Ansprüche, **dadurch gekennzeichnet, daß** ein nachzuweisender Analyt mit einem an einer festen Phase immobilisierten Fusionsprotein F1 und einem löslichen Fusionsprotein F2, welches markiert sein kann, in Kontakt gebracht wird, so daß ein detektierbarer Immunkomplex entsteht, **dadurch gekennzeichnet, daß** der zur Expression der Fusionsproteine eingesetzte Wirt ein Bakterium, beispielsweise E. coli ist und daß F1 weitgehend aus der unlöslichen Fraktion und F2 weitgehend aus der löslichen Fraktion des Bakterienlysats gewonnen wird.

11. Verfahren gemäß einem der vorhergehenden Ansprüche, **dadurch gekennzeichnet, daß** F1 im Vektor pSEM und F2 im Vektor pMAL™ exprimiert werden.

12. Verfahren gemäß einem der vorhergehenden Ansprüche, **dadurch gekennzeichnet, daß** der Analyt ein Antikörper ist oder die Analyten Antikörper sind.

13. Verfahren gemäß Anspruch 12, **dadurch gekennzeichnet, daß** die Antikörper gegen Viren, Bakterien, Parasiten, Allergene, Autoantigene, Arzneimittel oder Teile der vorstehend genannten Antigene gerichtet sind.

14. Verfahren gemäß Anspruch 12, **dadurch gekennzeichnet, daß** die Antikörper gegen HIV 1 und/oder HIV 2 oder T. pallidum gerichtet sind.

15. Testkit enthaltend mindestens zwei Bindungspartner gemäß einem der vorhergehenden Ansprüche sowie die zur Durchführung der darin beschriebenen Verfahren notwendigen weiteren Komponenten.

## Claims

1. An immunochemical method for qualitatively or quantitatively detecting an analyte in a sample, in which first and second binding partners are brought into contact with the analyte and the binding of the binding partners to the analyte is detected using known methods, which comprises preparing the two binding partners recombinantly in the same host using different vectors (V1 and V2) for expressing fusion proteins, with the first binding partner being expressed as fusion protein F1 in vector V1 and the second binding partner being expressed as fusion protein F2 in vector V2.

2. The method as claimed in claim 1, wherein at least 2 analytes are detected qualitatively or quantitatively, two binding partners are employed per analyte, and these two binding partners are prepared recombinantly, as fusion proteins, in the same host using different vectors for expressing foreign proteins, with it being possible to express binding partners of different analytes in different hosts.

3. The method as claimed in claim 1 or 2, wherein one or more of the binding partners is/are immobilized on a solid phase.

4. The method as claimed in claim 3, which is a double-antigen sandwich immunoassay method.

5. The method as claimed in one of the preceding claims, wherein the host is a eukaryotic organism.

6. The method as claimed in one of the preceding claims, wherein the host is a prokaryotic organism.

7. The method as claimed in claim 6, wherein the host is a bacterium.

8. The method as claimed in claim 7, wherein the bacterium is E. coli.

9. The method as claimed in one of the preceding. claims, wherein, within the context of the expression, one of the fusion proteins F1 or F2 is to a large extent obtained from the soluble fraction and the other fusion protein F1 or F2 in each case is to a large extent obtained from the insoluble fraction.

10. The method as claimed in one of the preceding claims, wherein an analyte to be detected is brought into contact with a fusion protein F1 which is immobilized on a solid phase and a soluble fusion protein F2 which can be labeled such that a detectable immune complex is produced, wherein the host which is employed for expressing the fusion proteins is a bacterium, for example E. coli, and F1 is to a large extent obtained from the insoluble fraction and F2 is to a large extent obtained from the soluble fraction of the bacterial lysate.

11. The method as claimed in one of the preceding claims, wherein F1 is expressed in the vector pSEM and F2 is expressed in the vector PMAL™.

12. The method as claimed in one of the preceding claims, wherein the analyte is an antibody or the analytes are antibodies.

13. The method as claimed in claim 12, wherein the antibodies are directed against viruses, bacteria, parasites, allergens, autoantigens or pharmaceuticals, or parts of the previously mentioned antigens.

14. The method as claimed in claim 12, wherein the antibodies are directed against HIV 1 and/or HIV 2 or T. pallidum.

15. A test kit which comprises at least two binding partners as claimed in one of the preceding claims and also the additional components which are required for carrying out the methods which are described therein.

## Revendications

1. Procédé immunochimique pour la détection qualitative ou quantitative d'un analyte dans un échantillon, dans lequel un premier partenaire de liaison et un second sont mis en contact avec l'analyte et la liaison des partenaires de liaison à l'analyte est détectée au moyen de procédés connus, **caractérisé en ce que** les deux partenaires de liaison sont produits par génie génétique dans le même hôte au moyen de vecteurs différents (V1 et V2) pour l'expression de protéines de fusion, le premier partenaire de liaison étant exprimé sous forme de protéine de fusion F1 dans le vecteur V1 et le second partenaire de liaison étant exprimé sous forme de protéine de fusion F2 dans le vecteur V2.

2. Procédé selon la revendication 1, **caractérisé en ce qu'**au moins 2 analytes sont détectés qualitativement ou quantitativement, **en ce qu'**on utilise deux partenaires de liaison par analyte et **en ce que** ces deux partenaires de liaison sont produits sous forme de protéines de fusion, par génie génétique, dans le même hôte au moyen de vecteurs différents, pour l'expression de protéines de fusion, des partenaires de liaison d'analytes différents pouvant être exprimés dans des hôtes différents.

3. Procédé selon la revendication 1 ou 2, **caractérisé en ce qu'**un ou plusieurs des partenaires de liaison est/sont immobilisé(s) sur une phase solide.

4. Procédé selon la revendication 3, **caractérisé en ce qu'**il s'agit d'un procédé de dosage immunologique en sandwich à double antigène.

5. Procédé selon l'une quelconque des revendications précédentes, **caractérisé en ce que** l'hôte est un organisme eucaryote.

6. Procédé selon l'une quelconque des revendications précédentes, **caractérisé en ce que** l'hôte est un organisme procaryote.

7. Procédé selon la revendication 6, dans lequel l'hôte est une bactérie.

8. Procédé selon la revendication 7, dans lequel la bactérie est *E. coli.*

9. Procédé selon l'une quelconque des revendications précédentes, **caractérisé en ce que** dans le cadre de l'expression on obtient l'une des protéines de fusion F1 ou F2 dans une large mesure à partir de la fraction soluble et l'autre protéine de fusion F1 ou F2 dans une large mesure à partir de la fraction insoluble.

10. Procédé selon l'une quelconque des revendications précédentes, **caractérisé en ce qu'**un analyte à détecter est mis en contact avec une protéine de fusion F1 immobilisée sur une phase solide et avec une protéine de fusion F2 soluble qui peut être marquée, de sorte qu'il en résulte un immunocomplexe détectable, **caractérisé en ce que** l'hôte utilisé pour l'expression des protéines de fusion est une bactérie, par exemple *E. coli*, et **en ce qu'**on obtient F1 dans une large mesure à partir de la fraction insoluble et F2 dans une large mesure à partir de la fraction soluble du lysat de bactéries.

11. Procédé selon l'une quelconque des revendications précédentes, **caractérisé en ce que** F1 est exprimée dans le vecteur pSEM et F2 est exprimée dans le vecteur pMAL™.

12. Procédé selon l'une quelconque des revendications précédentes, **caractérisé en ce que** l'analyte est un anticorps ou les analytes sont des anticorps.

13. Procédé selon la revendication 12, **caractérisé en ce que** les anticorps sont dirigés contre des virus, des bactéries, des parasites, des allergènes, des auto-antigènes, des médicaments ou des parties des antigènes précités.

14. Procédé selon la revendication 12, **caractérisé en ce que** les anticorps sont dirigés contre VIH 1 et/ou VIH 2 ou *T. pallidum*.

15. Nécessaire d'essai contenant au moins deux partenaires de liaison selon l'une quelconque des revendications précédentes, ainsi que les autres composants requis pour l'exécution des procédés décrits.
